(19) **Europäisches Patentamt**
**European Patent Office**
**Office européen des brevets**

(11) **EP 2 222 181 B1**

(12) **EUROPEAN PATENT SPECIFICATION**

(45) Date of publication and mention
of the grant of the patent:
**12.10.2011 Bulletin 2011/41**

(51) Int Cl.:
*A23F 3/06* [(2006.01)]     *A23F 3/08* [(2006.01)]
*A23F 3/16* [(2006.01)]     *A23F 3/18* [(2006.01)]
*A23F 3/30* [(2006.01)]

(21) Application number: **08847503.3**

(22) Date of filing: **30.10.2008**

(86) International application number:
**PCT/EP2008/064717**

(87) International publication number:
**WO 2009/059927 (14.05.2009 Gazette 2009/20)**

(54) **PROCESS FOR MANUFACTURING TEA PRODUCTS**

VERFAHREN ZUR HERSTELLUNG VON TEEPRODUKTEN

PROCÉDÉ DE PRÉPARATION DE PRODUITS À BASE DE THÉ

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **05.11.2007 EP 07119988
05.11.2007 EP 07119984
12.11.2007 EP 07120448
12.11.2007 EP 07120447
19.12.2007 EP 07123586
07.02.2008 EP 08151155
02.10.2008 EP 08165775
02.10.2008 EP 08165776**

(43) Date of publication of application:
**01.09.2010 Bulletin 2010/35**

(73) Proprietors:
• **Unilever PLC, A Company Registered in England
and
Wales under Company no. 41424
London EC4Y 0DY (GB)**
Designated Contracting States:
**CY GB IE MT**
• **Unilever N.V.
3013 AL Rotterdam (NL)**
Designated Contracting States:
**AT BE BG CH CZ DE DK EE ES FI FR GR HR HU
IS IT LI LT LU LV MC NL NO PL PT RO SE SI SK TR**

(72) Inventors:
• **COLLIVER, Steven, Peter
Sharnbrook Bedfordshire MK44 1LQ (GB)**
• **DOWNIE, Andrew, Lee
Sharnbrook Bedfordshire MK44 1LQ (GB)**
• **SHARP, David, George
Sharnbrook Bedfordshire MK44 1LQ (GB)**
• **YOU, Xiaoqing
Sharnbrook Bedfordshire MK44 1LQ (GB)**

(74) Representative: **Keenan, Robert Daniel
Unilever Patent Group
Colworth House
Sharnbrook
Bedford
Bedfordshire MK44 1LQ (GB)**

(56) References cited:
**EP-A- 0 167 399**    **WO-A-00/47056**
**WO-A-01/82713**    **WO-A-99/40799**
**WO-A-03/022066**    **WO-A-2008/040627**
**GB-A- 968 423**    **US-A1- 2007 071 870**

Printed by Jouve, 75001 PARIS (FR)

## EP 2 222 181 B1

**Description**

### TECHNICAL FIELD OF THE INVENTION

The present invention relates to a process for manufacturing tea products. In particular, the present invention relates to a process for manufacturing tea products from juice expressed from fermented tea leaves.

### BACKGROUND TO THE INVENTION

*Beverages based on the tea plant *(Camellia sinensis)* have been popular throughout the world for many hundreds of years. Traditionally such beverages are produced by infusing leaf tea in hot water.

Although many consumers still enjoy beverages made from leaf tea, it is becoming increasingly popular to enjoy tea beverages prepared in more convenient ways. For example, tea beverages can be prepared from instant powders which are free from insoluble leaf tea and so dissolve rapidly and completely on contact with hot water. These powder products are usually manufactured by a process comprising extracting leaf tea with water and drying the resulting extract. Also popular are packaged ready-to-drink beverages which contain dissolved tea solids. Such ready-to-drink teas are usually manufactured from instant powders such as those described above or directly from extraction of tea leaf.

Packaged ready-to-drink tea beverages can sometimes be unstable. In particular, such beverages can often develop haze on storage. This haze formation has been attributed, at least in part, to the poor cold-water solubility of polyphenol species present in the tea solids and which comprise a gallate moiety. Such gallated polyphenols can also bring unwanted bitterness or astringency to tea beverages. Thus technologies have been developed for decreasing the amount of gallated polyphenols in tea solids.

US 4,051,264 (Thomas J. Lipton, Inc.) discloses treatment of fresh green tea with tannase in order to improve the cold water solubility of tea solids. Tannase is an enzyme which hydrolyzes gallate ester linkages. A drawback with using tannase treatment to decrease the amount of gallated polyphenols is that the gallic acid released during desterification can negatively impact on the flavour of beverages made from the treated tea solids.

Consumers are also increasingly interested in foods and beverages which have a low caffeine content, owing to the perceived health risks of a high caffeine diet. Furthermore, caffeine is known to add to the bitterness of tea beverages. Thus there have been many efforts to manufacture low-caffeine tea products, usually using processes employing extraction of caffeine from tea leaves using a solvent such as methylene chloride, ethyl acetate or carbon dioxide.

US 2007/0231445 A1 discloses a method for decaffeinating tea using carbon dioxide compressed to a pressure greater than 50 and up to 100 MPa. A drawback of such decaffeination processes is that the use of non-aqueous solvents is not only unfriendly to the environment, but can also result in removal of valuable compounds from tea other than caffeine.

Surprisingly we have found that the juice expressed from tea leaves under certain conditions is naturally low in gallated polyphenols and/or caffeine. In particular we have found that such juice can be expressed in high yield from leaves having undergone a specific degree of fermentation.

GB 1,284,721 (FINLIP PRODUCTS LIMITED) discloses a process wherein an extract of tea solids which is soluble in cold water is obtained wholly or partially by one or more pressings of tea leaves, followed by at least one further extraction of the tea leaves with hot water, the said hot water extract being separately subjected to an oxidative solubilising process, and thereafter combining the said extracts to provide a tea product. This document does not disclose the amount of juice expressed from the tea leaves. Furthermore, the tea products prepared by this process are likely to contain substantial levels of gallated polyphenols and/or caffeine as these compounds would be extracted by the hot water extraction.

GB 968,423 (A.M.H. Bake) discloses a method of producing a stable tea juice from fresh tea leaf that has been subjected to the stages of withering, bruising, twisting, fermenting, killing of enzymes, excluding drying, or a selected sequence of these stages, consisting in separating the tea juice from the thus processed leaf in its still moist state. This document does not disclose the amount of juice expressed from the tea leaves. Furthermore, this document does not disclose the specific degree of fermentation required to produce tea juice with the exceptional qualities of the tea juice of the present invention.

US 2007/071870 A (CONOPCO INC) discloses a process for making black or green tea with the floral aroma of oolong tea. The process utilises tumbling to physically wound the leaves to initiate the oolong aromas and does not involve a traditional solar wither step.

WO 2008/040627 (Unilever) discloses a palatable green leaf tea from *Camellia sinensis* var. assamica.

WO 99/40799 (Unilever) discloses a method for manufacturing a black leaf tea that resembles orthodox processed black tea but infuses like CTC processed black tea.

EP 0 167 399 A (GENERAL FOODS CORP) discloses contacting fermented, but unfired, green tea leaf material with a decaffeination fluid in order to effect removal of caffeine from the tea.

WO 2000/47056 (Unilever) discloses a cold water infusible tea leaf product which brews in cold water to produce an iced tea beverage with the color and flavor of iced teas prepared by hot brewing methods.

WO 2001/82713 (Unilever) discloses a process for manufacturing black leaf tea that looks and feels like orthodox processed tea but has the liquor characteristics of a fuller fermented CTC processed tea.

WO 2003/022066 (Unilever) discloses a process to make tea with higher yields of a desired size comprising the steps of: (a) withering green leaf tea (b) size reduction (c) fermentation (d) extrusion of the size reduced or fermented tea leaf mass through holes in a perforated body (e) cutting the extrudate (f) drying.

**TESTS AND DEFINITIONS**

Tea

"Tea" for the purposes of the present invention means material from *Camellia sinensis* var. *sinensis* and/or *Camellia sinensis* var. *assamica*. Especially preferred is material from var. *assamica* as this has a higher level of tea actives than var. *sinensis.*

"Leaf tea" for the purposes of this invention means a tea product that contains tea leaves and/or stem in an uninfused form, and that has been dried to a moisture content of less than 30% by weight, and usually has a water content in the range 1 to 10% by weight (i.e. "made tea").

"Green tea" refers to substantially unfermented tea. "Black tea" refers to substantially fermented tea. "Oolong tea" refers to partially fermented tea.

"Fermentation" refers to the oxidative and hydrolytic process that tea undergoes when certain endogenous enzymes and substrates are brought together, e.g., by mechanical disruption of the cells by maceration of the leaves. During this process colourless catechins in the leaves are converted to a complex mixture of yellow and orange to dark-brown polyphenolic substances.

"Fresh tea leaves" refers to tea leaves and/or stem that have never been dried to a water content of less than 30% by weight, and usually have a water content in the range 60 to 90%.

"Dhool" refers to macerated fresh tea leaves.

Expressing Juice

As used herein the term "expressing juice" refers to squeezing out juice from dhool using physical force, as opposed to extraction of tea solids with the use of a solvent. Thus the term "expressing" encompasses such means as squeezing, pressing, wringing, spinning and extruding. It is possible that a small amount of solvent (e.g. water) is added to the dhool during the expression step. However, in order to prevent significant extraction of tea solids by the solvent, the moisture content of the dhool during expression is that of fresh tea leaves as defined hereinabove. In other words, during the expression step, the moisture content of the dhool is between 30 and 90% by weight, more preferably between 60 and 90%. It is also preferred that the fresh leaves and/or dhool are not contacted with non-aqueous solvent (e.g. alcohols) prior to or during expression, owing to the environmental & economic problems associated with such solvents.

Beverage

As used herein the term "beverage" refers to a substantially aqueous drinkable composition suitable for human consumption.

Leaf Size and Grade

For the purposes of the present invention, leaf particle size is characterised by sieve mesh size using the following convention:

- Tyler mesh sizes are used throughout.
- A "+" before the sieve mesh indicates the particles are retained by the sieve.
- A "-" before the sieve mesh indicates the particles pass through the sieve.

For example, if the particle size is described as -5 +20 mesh, then the particles will pass through a 5 mesh sieve (particles smaller than 4.0 mm) and be retained by a 20 mesh sieve (particles larger than 841 $\mu$m).

Leaf particle size may additionally or alternatively be characterized using the grades listed in the international standard ISO 6078-1982. These grades are discussed in detail in our European patent specification EP 1 365 657 B1 (especially paragraph [0041] and Table 2) which is hereby incorporated by reference.

Catechins

As used herein the term "catechins" is used as a generic term for catechin, gallocatechin, catechin gallate, gallocatechin gallate, epicatechin, epigallocatechin, epicatechin gallate, epigallocatechin gallate, and mixtures thereof. The catechins are sometimes referred to using the following shorthand notation: C for catechin, GC for gallocatechin, CG for catechin gallate, GCG for gallocatechin gallate, EC for epicatechin, EGC for epigallocatechin, ECG for epicatechin gallate, and EGCG for epigallocatechin gallate. The term "gallated catechins" is used as a generic term for CG, ECG, GCG, EGCG and mixtures thereof.

Theaflavins

As used herein the term "theaflavins" is used as a generic term for theaflavin, theaflavin-3-gallate, theaflavin-3'-gallate, theaflavin-3,3'-digallate and mixtures thereof. The structures of these compounds are well-known (see, for example, structures xi-xiv in Chapter 17 of "Tea - Cultivation to consumption", K.C. Willson and M.N. Clifford (Eds), 1992, Chapman & Hall, London, pp.555-601). The theaflavins are sometimes referred to using the shorthand notation TF1-TF4 wherein TF1 is theaflavin, TF2 is theaflavin-3-gallate, TF3 is theaflavin-3'-gallate and TF4 is theaflavin-3,3'-digallate (or simply "theaflavin digallate"). The term "gallated theaflavins" is used as a generic term for TF2, TF3, TF4 and mixtures thereof.

Determination of Catechins and Caffeine in Tea Leaves or Dhool

For fresh tea leaves, the leaves are steamed to prevent fermentation and then dried to yield green leaf tea. For dhool, the dhool is fired to arrest fermentation and yield leaf tea. The amounts of catechins and caffeine in the leaf tea are then determined simultaneously by reverse-phase HPLC as follows.

*Sample Preparation*

1. Grind the leaf tea using a Cyclotech™ 1093 sample mill (FOSS Ltd, Warrington, Cheshire, UK) fitted with a 0.5 µm screen, until a fine powder is achieved.
2. Weigh accurately approximately 200 mg of the ground tea into an extraction tube, and record the mass.
3. Warm at least 20 ml of a methanol-water solution (70% v/v methanol in distilled water) to 70°C.
4. Add 5 ml of the hot methanol-water solution to the extraction tube. Gently mix the methanol-water and tea material on a vortex mixer; place in a water bath at 70°C for 5 minutes; mix again and then place in a water bath at 70°C for a further 5 minutes.
5. Gently mix the methanol-water and tea material on a vortex mixer again and then allow too cool for a 10 minutes at an air temperature of 20°C.
6. Centrifuge the extraction tube at a relative centrifugal force (RCF) of 2900 g for 10 minutes.
7. The extraction tube should now contain a liquid supernatant on top of a plug of tea material. Carefully decant supernatant into a clean graduated test tube.
8. Add 5 ml of the hot methanol-water solution to the plug in the extraction tube. Gently mix the methanol-water and tea material on a vortex mixer; place in a water bath at 70°C for 5 minutes; mix again and then place in a water bath at 70°C for a further 5 minutes.
9. Gently mix the methanol-water and tea material on a vortex mixer again and then allow too cool for a 10 minutes at an air temperature of 20°C.
10. Centrifuge the extraction tube at a RCF of 2900 g for 10 minutes.
11. The extraction tube should now contain a liquid supernatant on top of a plug of tea material. Carefully decant supernatant into the graduated test tube containing the supernatant from step 7.
12. Make up the pooled supernatants to 10 ml with the methanol-water solution.
13. Add 1 ml of a solution of 2.5 mg/ml EDTA and 2.5 mg/ml ascorbic acid in distilled water to the graduated test tube.
14. Dilute 1 part of the pooled supernatant mixture with 4 parts (by volume) of 10% acetonitrile stabiliser solution (10% v/v acetonitrile, 0.25 mg/ml ascorbic acid and 0.25 mg/ml EDTA in distilled water).
15. Decant the diluted pooled supernatant mixture into microcentrifuge tubes and centrifuge in a bench top centrifuge at a RCF of 14000 g for 10 minutes.

*HPLC Analysis conditions*

| Column: | Luna Phenyl hexyl 5μ, 250 x 4.60 mm |
|---|---|
| Flow rate: | 1 ml/min |
| Oven temperature: | 30°C |

| Solvents: | A: 2% acetic acid in acetonitrile |
|---|---|
| | B: 2% acetic acid and 0.02 mg/ml EDTA in water |

**Injection volume:** 10 μl

**Gradient:**

| Time | % Solvent A | % Solvent B | Step |
|---|---|---|---|
| 0 to 10 min | 5 | 95 | Isocratic |
| 10 to 40 min | 5 - 18 | 95 - 85 | Linear gradient |
| 40 to 50 min | 18 | 82 | Isocratic |
| 50 to 55 min | 50 | 50 | Wash |
| 55 to 75 min | 5 | 95 | Isocratic |

**Quantification:** Peak area relative to a calibration curve constructed daily. Calibration curve is constructed from caffeine and the concentration of catechins is calculated using the relative response factors of the individual catechins to caffeine (from the ISO catechin method - ISO/CD 14502-2). Individual caffeine standards (Sigma, Poole, Dorset, UK) are used as peak identification markers.

Determination of Catechins and Caffeine in Juice and Beverages The amounts of catechins and caffeine in a liquid sample are determined simultaneously by reverse-phase HPLC as follows:

*Sample Preparation*

1. 9 ml of the sample are taken and 1.12 ml of acetonitrile added, along with 1.12 ml of a solution of 2.5 mg/ml EDTA and 2.5 mg/ml ascorbic acid in distilled water.
2. The resulting solution is then decanted into microcentrifuge tubes and centrifuged at a RCF of 14000 g for 10 minutes.

*HPLC Analysis conditions*

The HPLC analysis conditions are identical to those given above for the tea leaves.

Determination of Theaflavins in Juice and Beverages

Reversed-phase high performance liquid chromatography is used to quantify the amount of theaflavins in a liquid sample as follows:

*Sample Preparation*

1. 2 parts by weight of acetonitrile and 1 part by weight of a stabilising solution of 25 mg/ml EDTA and 25 mg/ml ascorbic acid in distilled water is added to 8 parts by weight of sample.

2. The diluted sample is then decanted into microcentrifuge tubes and centrifuged at a relative centrifugal force (RCF) of 14000 g for 10 minutes.

*HPLC Analysis conditions*

| | |
|---|---|
| **Column:** | Hypersil C18, 3μ, 100 x 4.60mm |
| **Flow rate:** | 1.8 ml/min |
| **Oven temperature:** | 30°C |

| | |
|---|---|
| **Solvents:** | A: 2% acetic acid in acetonitrile |
| | B: 2% acetic acid in water |

| | |
|---|---|
| **Injection volume:** | 10 μl |
| **Gradient:** | Isocratic at 20% A and 80% B. |

**Quantification:** The catechins are eluted at the beginning of the chromatogram in a broad unresolved peak and the theaflavins are eluted between 5-15 min. Detection is at 274 nm. Peak area is measured relative to a calibration curve constructed daily. The calibration curve is constructed from a series of solutions containing known amounts of a tea extract previously analysed against pure theaflavin standards.

Determination of Theanine in Juice and Beverages

The amount of theanine in a liquid sample is determined by reversed phase HPLC chromatography using fluorimetric detection following post-column derivatisation with o-pthalaldehyde.

*Sample Preparation*

The sample is diluted with de-ionised water (25°C) in a weight ratio of sample:water of 1:10.

*HPLC Analysis conditions*

| | |
|---|---|
| **Column:** | Hypersil HyPURITY Elite™ C18, 5μ, 150mm x 4.6cm |
| **Flow rate:** | 1 ml/min |
| **Oven temperature:** | 35°C |

| | |
|---|---|
| **Solvents:** | A: 5 mM pentadecafluorooctanoic acid in water |
| | B: 5 mM pentadecafluorooctanoic acid in acetonitrile |

| Gradient: | | |
|---|---|---|
| Time (min) | % Solvent A | % Solvent B |
| 0 | 85 | 15 |
| 8 | 85 | 15 |
| 10 | 80 | 20 |
| 11 | 10 | 90 |

(continued)

| Time (min) | % Solvent A | % Solvent B |
| --- | --- | --- |
| 14 | 10 | 90 |
| 15 | 85 | 15 |
| 31 | 85 | 15 |

**Quantification:** The eluant from the column is fed into a low dead-volume 3-way junction and mixed with the o-Pthalaldehyde reagent in a 1:1 ratio, the o-Pthalaldehyde reagent being pumped at 1 ml/minute by the isocratic pump. (The o-Pthalaldehyde reagent is 1.0 g/l o-Pthalaldehyde, 5 ml/l methanol, 2 ml/l Brij 35 and 3 ml/l 2-mercaptoethanol in pH 10 borate buffer.) Fluorescence detection is: Excitation = 340 nm and Emission = 425 nm. Peak area relative to a calibration curve constructed daily is used for quantification. The calibration curve is constructed from dilutions of a standard solution of Suntheanine™ (Taiyo KK).

Determination of Total Polyphenols

The total polyphenol content of a sample is determined using the Folin-Ciocalteu method as detailed in the International Standard published by the International Organization for Standardization as ISO 14502-1:2005(E).

**SUMMARY OF THE INVENTION**

We have surprisingly found that the amount of caffeine in tea juice expressed from dhool decreases with the degree of fermentation. Furthermore we have found that tea juice typically has a lower proportion of gallated theaflavins compared with conventional tea extracts and that the proportion of gallated theaflavins in the juice also varies with the degree of fermentation before expression. In particular, we have found that juice expressed from dhool which has been fermented for sufficient time to oxidise at least 50% of the catechins in the tea leaves is particularly low in caffeine and/or gallated theaflavins.

Thus in a first aspect, the present invention provides a process comprising the steps of:

a) providing fresh tea leaves comprising catechins;
b) macerating the fresh tea leaves thereby to produce dhool;
c) fermenting the dhool for a fermentation time ($t_F$) sufficient to reduce the content of catechins in the dhool to less than 50% of the content of catechins in the fresh tea leaves on a dry weight basis; and then
d) expressing juice from the fermented dhool thereby to produce leaf residue and tea juice, wherein the amount of expressed juice is at least 50 ml per kg of the fresh tea leaves.

In a second aspect, the present invention provides tea juice obtained and/or obtainable by the process and in a third aspect a beverage obtained and/or obtainable by diluting the tea juice. Such juice and beverages will have a low level of gallated theaflavins and/or a low level of caffeine and thus may be more stable and/or less bitter than tea juices produced by alternative processes.

A convenient indication of the proportion of gallated theaflavins is the ratio of TF1 to TF4 and a convenient indication of the caffeine content is the ratio of theanine to caffeine.

**DETAILED DESCRIPTION**

Providing Fresh Tea Leaves

Step (a) of the process of the invention comprises providing fresh tea leaves comprising catechins.

It is particularly preferred that the fresh tea leaves comprise material from var. *assamica* as this variety naturally has a high level of tea actives and so leads to a high level of actives in the leaf residue even after removal of the juice. Most preferably the fresh leaves are fresh leaves from var. *assamica*.

The fresh tea leaves are preferably provided in freshly plucked form, i.e. without any further processing. The fresh tea leaves preferably comprise actively growing buds, e.g. in the form of the first two or three leaves together with the unopened bud (so-called "two-and-a-bud" and/or "three-and-a-bud" material).

The fresh tea leaves may be withered prior to step (b). If so, the tea leaves are typically withered for about 12 to 36 hours. Withering allows certain chemical and biochemical changes to occur and also reduces the moisture content of the leaves to around 35 to 70%. The biochemical and/or chemical changes taking place during withering may increase the yield of the volatile flavour compounds in tea.

The present invention is found to work well with fresh leaves with or without any special pre-treatment. Thus it is preferred that the leaves have not undergone a freeze-thaw process prior to step (b) and/or step (d).

Furthermore, in order to allow fermentation to occur without the use of exogenous enzymes, it is preferred that the fresh tea leaves have not been heat-treated in order to deactivate the endogenous fermentation enzymes.

Macerating the Fresh Tea Leaves

Step (b) of the process of the invention comprises macerating the fresh tea leaves thereby to produce dhool.

Maceration involves wounding the leaves e.g. by rolling and/or crushing the leaves i.e. to break down the plant tissue structure. In black tea manufacture this has the effect of liberating fermentable substrates and fermenting enzymes from within the plant cells and tissue. The maceration is preferably achieved by passing the fresh tea leaves through a cutting machine. Thus for the purpose of the invention the fresh tea leaves may be macerated by a maceration process using, for example, a CTC machine, rotorvane, ball mill, grinder, hammer mill, Lawri tea processor, Legg cutting machine, or tea rollers as in orthodox tea processing. Combinations of these maceration processes may also be used.

Fermenting the Dhool

Step (c) of the process of the invention comprises fermenting the dhool.

The degree of fermentation is conveniently judged by the proportion of oxidised catechins. In particular, one can measure a quantity, $C_0$, which is the amount of catechins in the fresh tea leaves prior to maceration in percent by dry weight of the fresh leaves. One can then measure a second quantity, $C_F$, which is the amount of catechins in the dhool after a given fermentation time, $t_F$, in percent by dry weight of the dhool. One can then use these values to calculate the degree of fermentation, R, as the content of catechins in the dhool at $t_F$ as a percentage of the content of catechins in the fresh tea leaves prior to maceration on a dry weight basis. In other words, the degree of fermentation can be calculated as follows:

$$R(\%) = 100 C_F / C_0,$$

such that for negligible fermentation R = 100% and for complete fermentation R = 0%

We have found that fermentation for a time ($t_F$) sufficient to reduce the content of catechins in the dhool to less than 50% of the content of catechins in the fresh tea leaves prior to maceration on a dry weight basis (i.e. R < 50%) results in significant and surprising changes in the composition of the juice expressed from the dhool. In particular, the greater the degree of fermentation (lower R), the lower the proportion of gallated theaflavins and/or caffeine is found in the expressed juice. Preferably, $t_F$ is sufficient to reduce the amount of catechins in the dhool to less than 40% (i.e. R < 40%), more preferably less than 30%, and most preferably from 25 to 0% of the content of catechins in the fresh tea leaves prior to maceration on a dry weight basis.

The exact time required to produce the desired degree of fermentation will depend, amongst other things, on the temperature of the dhool, the degree of maceration of the dhool and the supply of oxygen to the dhool. Typically, however, $t_F$ is at least 1 hour, more preferably at least 1.5 hours, more preferably still at least 1.75 hors and most preferably from 2 to 24 hours.

The preferred fermentation temperature is from 10 to 40°C, more preferably from 15 to 25°C. Too low a temperature results in a slow rate of fermentation whilst too high a temperature may result in deactivation of oxidative enzymes and/or generation of unwanted reaction products.

Expression of Juice

Step (d) of the process of the invention comprises expressing juice from the fermented dhool thereby to produce leaf residue and tea juice, wherein the amount of expressed juice is at least 50 ml per kg of the fresh tea leaves.

If the amount of juice expressed is too low then it becomes difficult to separate the juice from the leaf residue and/or leads to an inefficient process. Thus it is preferred that the amount of expressed juice is at least 100 ml per kg of the fresh tea leaves, more preferably at least 150 ml, more preferably still at least 175 ml and most preferably at least 200 ml. When referring to the volume of juice expressed per unit mass of tea leaves it should be noted that the mass of the tea leaves is expressed on an "as is" basis and not a dry weight basis. Thus the mass includes any moisture in the leaves.

It is also advantageous to limit the amount of juice expressed as this limits damage to the residual leaf and allows it to be used to manufacture tea products of at least conventional quality. Thus it is preferred that the amount of expressed juice is less than 800 ml per kg of fresh leaves, more preferably less than 500 ml, more preferably still less than 300 ml and most preferably less than 275 ml.

The expression step can be achieved in any convenient way so long as it allows for separation of the tea juice from the leaf residue and results in the required quantity of juice. The machinery used to express the juice may, for example, include a hydraulic press, a pneumatic press, a screw press, a belt press, an extruder or a combination thereof.

The juice may be obtained from the dhool in a single pressing or in multiple pressings of the dhool. Preferably the juice is obtained from a single pressing as this allows for a simple and rapid process.

In order to minimise degradation of the valuable tea compounds, it is preferred that the expression step is performed at ambient temperature. For example, the dhool temperature may be from 5 to 40°C, more preferably 10 to 30°C.

The time and pressure used in the expression step can be varied to yield the required amount of juice. Typically, however, the pressures applied to express the juice will range from 0.5 MPa (73 psi) to 10 MPa (1450 psi). The time over which the pressure is applied will typically range from 1 s to 1 hour, more preferably from 10 s to 20 minutes and most preferably from 30 s to 5 minutes.

Processing the Leaf Residue

In order to maximise the efficiency of the process it is preferred that the leaf residue is not discarded but is further processed to produce a commercially viable product, such as leaf tea and/or tea extract. In a particularly preferred embodiment, the process comprises an additional step (e) wherein the leaf residue is processed to produce leaf tea.

The leaf residue may be processed to produce black leaf tea or oolong leaf tea, more preferably black leaf tea.

The manufacturing processes of black leaf tea and oolong leaf tea are well known and suitable processes are described, for example, in "Tea: Cultivation to Consumption", K.C. Willson and M.N. Clifford (Eds), 1st Edn, 1992, Chapman & Hall (London), Chapters 13 and 14.

A step common to manufacture of all leaf teas is a drying step. In the case of oolong and black leaf tea, the drying step usually also serves to deactivate the fermentation enzymes. Efficient drying requires high temperatures and so it is preferred that step (e) of the process comprises drying the leaf residue at a temperature of at least 75°C, more preferably at least 90°C.

It is preferred that step (e) comprises sorting the leaf tea, preferably after drying, to achieve a particle size of at least 35 mesh. More preferably the leaf tea is sorted to achieve a particle size of from 30 mesh to 3 mesh. Alternatively or additionally, the leaf tea may be sorted to achieve a leaf tea grade of Pekoe Fannings (PF) grade or larger, more preferably Orange Fannings (OF) or larger and most preferably Broken Orange Pekoe Fannings (BOPF) or larger.

Processing the Juice

Tea juice separated from the leaf residue typically has a high content of water-soluble tea solids and is a valuable raw material for producing tea products.

Owing to expression after the specified degree of fermentation, the juice is at least partially fermented. Thus the juice may be used to produce an oolong tea product or a black tea product, most preferably a black tea product.

In order to preserve the specific composition of the juice, especially in terms of gallated polyphenols and/or caffeine, it is preferred that if the tea juice is used to make a tea product then the tea juice is substantially the only source of tea solids in the tea product

*Diluting to Make a Beverage*

In one embodiment the tea juice is diluted to produce a beverage. A suitable process is described, for example, in CN 1 718 030 A (LANCANGJIANG BEER ENTPR GROUP).

The juice is preferably diluted with an aqueous medium, preferably water. The beverage typically comprises at least 85% water, more preferably at least 90%, optimally between 95 and 99.9% by weight of the beverage.

Because the juice is relatively rich in tea solids, it can be diluted many-fold whilst still imparting tea-qualities to the resulting beverage. Preferably, therefore, the juice is diluted by at least a factor of 2 to produce the beverage (i.e. 1 part of juice is combined with 1 part diluent by weight). More preferably the juice is diluted by a factor of at least 5 (i.e. 1 part of juice is combined with 4 parts diluent by weight) and most preferably by a factor of at least 7.

The juice can be used to make concentrated beverages with high levels of tea solids. For example, the juice may be diluted by a factor of less than 50, more preferably less than 25 and most preferably less than 15.

The mass of a single serve of the beverage may be, for example, less than 600 g, more preferably less than 350 g, more preferably still less than 250 g and most preferably from 20 to 150 g.

The pH of the beverage may, for example, be from 2.5 to 8, more preferably 3 to 6, most preferably from 3.5 to 6. The beverage may comprise a food grade acid and/or salt thereof such as citric, malic, ascorbic acid or a mixture thereof.

The beverage preferably comprises at least one nutrient selected from carbohydrate, protein, fat, vitamins, minerals and mixtures thereof. The beverage may be low calorie (e.g. have an energy content of less than 100 kCal per 100 g of the beverage) or may have a high calorie content (e.g. have an energy content of more than 100 kCal per 100 g of the beverage, preferably between 150 and 1000 kCal). It is most preferred that the beverage is very low calorie such that a single serving has a total energy content of less than 5 kCal, more preferably still less than 1 kcal.

The beverage may also comprise any of salt, sweetener, flavours, colours, preservatives, antioxidants or a mixture thereof.

The beverage is preferably packaged. The package will typically be a bottle, can, carton or pouch.

The beverage is preferably sanitised e.g. by pasteurisation or sterilisation.

*Drying the Juice*

In one embodiment the tea juice is dried to produce a liquid concentrate or powder. Preferably the juice is dried to a moisture content of less than 80% by weight, more preferably less than 50% by weight, more preferably still less than 30% by weight and most preferably less than 10% by weight. Any suitable drying process may be used including spray drying, freeze drying, oven drying, tray drying, vacuum drying or a combination thereof.

The concentrate or powder may, for example, be diluted or dissolved to produce a beverage, used as a food additive and/or used as a starting material for producing other tea-derived materials.

The Tea Juice and Beverages Prepared Therefrom

The present invention provides tea juice obtained and/or obtainable by the process as well as beverages obtained and/or obtainable by diluting the tea juice. Such juice and beverages will have a low level of gallated theaflavins and/or a low level of caffeine and thus may be more stable and/or less bitter than tea juices produced by alternative processes. Although the beverage is preferably directly obtained by diluting the liquid juice, it may be obtained by dissolving dried juice.

The tea juice will typically have a total solids content of from 4 to 12% by weight, more preferably from 6 to 10%.

The beverage will typically comprise tea solids in an amount of from 0.001 to 5% by weight of the beverage, more preferably 0.01 to 3% by weight and most preferably 0.1 to 1% by weight

Owing to the fermentation process employed before expression, the tea juice or beverage will typically comprise less than 50% catechins by weight of the total polyphenols in the juice and/or beverage, more preferably less than 40%, more preferably still less than 30%, and most preferably from 25 to 0%. Additionally or alternatively the juice or beverage will be black tea juice or a black tea beverage.

The juice or beverage will typically comprise a relatively low proportion of gallated polyphenols. Conveniently this can be represented by the weight ratio of theaflavin (TF1) to theaflavin digallate (TF4). Preferably TF1/TF4 is at least 2.0, more preferably at least 3.0, more preferably still at least 3.2 and most preferably from 3.5 to 5.0. Additionally or alternatively the amount of TF1 in the total theaflavins in the juice or beverage is preferably at least 40% by weight, more preferably at least 42% by weight and most preferably from 45 to 60%.

The juice or beverage will typically comprise a relatively low content of caffeine. Conveniently this can be represented by the weight ratio of theanine to caffeine. Preferably the weight ratio of theanine to caffeine is greater than 0.7, more preferably at least 0.9, more preferably still at least 1.0 and most preferably from 1.3 to 5.0.

The juice will typically comprise less than 4 mg/ml caffeine, more preferably less than 3 mg/ml and most preferably from 0.5 to 2 mg/ml.

The beverage will typically comprise less than 0.10 mg/ml caffeine, more preferably less than 0.08 mg/ml, more preferably still less than 0.07 mg/ml and most preferably from 0.01 to 0.06 mg/ml.

The Composition

The present invention provides a composition which provides the health benefits of theaflavins without unwanted bitterness from large proportions of caffeine and/or gallated thealfavins.

The composition comprising theaflavins, theanine and caffeine, wherein the theaflavins comprise theaflavin (TF1) and theaflavin digallate (TF4), and wherein:

- the weight ratio of theaflavin to theaflavin digallate (TF1/TF4) is at least 2.0; and
- the weight ratio of theanine to caffeine is greater than 0.7.

Preferably the composition has black tea character. Thus it is preferred that the composition comprise less than 50% catechins by weight of the total polyphenols in the composition, more preferably less than 40%, more preferably still less than 30%, and most preferably from 25 to 0%. Additionally or alternatively the theaflavins in the composition are preferably derived from black tea.

Preferably the weight ratio TF1/TF4 in the composition is at least 3.0, more preferably at least 3.2 and most preferably from 3.5 to 5.0. Additionally or alternatively the amount of TF1 in the total theaflavins in the composition is preferably at least 40% by weight, more preferably at least 42% by weight and most preferably from 45 to 60%.

The composition comprises a relatively low content of caffeine. The weight ratio of theanine to caffeine is greater than 0.7, more preferably at least 0.9, more preferably still at least 1.0 and most preferably from 1.3 to 5.0.

## EXAMPLES

The present invention will be further described with reference to the following examples.

Example 1

This Example demonstrates the effect of the degree of fermentation of dhool on the composition of juice expressed from the dhool.

*Fresh Leaves*

Fresh Kenyan tea leaves (two leaves and a bud) of *Camellia sinensis* var. *assamica* which had not been withered were used. The catechin content of the unmacerated leaves was about 15% by weight.

*Preparation and Fermentation of Dhool*

The fresh tea leaves were chopped using a vegetable cutter before being fed twice through a CTC (cut, tear, curl) machine (Rollers set at six teeth per inch with speeds of 1000 and 100 rpm respectively). The fresh dhool was then fermented for 2 hours at a temperature of 25°C using a Teacraft™ fermentation unit (0.5°C wet bulb depression, 90% R.H.). Samples of dhool were taken directly after maceration, after 1 hour of fermentation and at the end of fermentation in order to determine the amount of catechins in the dhool at these times. Each sample was dried in a fluid bed drier immediately after collection to arrest fermentation and produce leaf tea.

*Production of Juice*

Immediately after fermentation, a portion of dhool was pressed to yield substantially unfermented juice (Juice A). A second portion of dhool was removed from the fermenter after 1 hour of fermentation and pressed to yield 1 hour fermented juice (Juice B). The remainder of the dhool was pressed to yield 2 hour fermented juice (Juice C).

Pressing was done using a hydraulic press (5 Tonnes applied to a 500 g mass of fermented leaf inside a cylinder of diameter 160 mm, resulting in a downward pressure of 354 psi (2.44 MPa)) to express black tea juice. The tea juice was immediately centrifuged for 20 minutes (10000 g at 3°C) and the supernatant was then filter sterilised using a Nalgene™ filtration unit fitted with a 0.2 μm filter.

Details of the juice processing are shown in table 1.

TABLE 1

| Juice | Catechin content of dhool (wt%) | % Catechins Remaining in Dhool = R | Amount of Juice Expressed (ml kg$^{-1}$) | Total Solids of Juice (wt%) |
|-------|-------|-------|-------|-------|
| A | 13.6 | 91 | 170 | 9.3 |
| B | 4.5 | 30 | 250 | 6.4 |
| C | 3.3 | 22 | 252 | 6.1 |

*Production of Reference Infusion*

A portion of the dhool which had been fermented for 2 hours but which had not been pressed was broken up by hand and then dried using a fluidized bed drier (ten minutes at an inlet air temperature of 120°C, followed by ten minutes at an inlet air temperature of 90°C) to obtain a made black tea with moisture content of 3% by weight. An infusion of 2 g of this leaf tea was prepared by infusing 2 g of the tea in 200 ml freshly boiled water for 2 minutes.

*Results*

Table 2 shows the chemical composition of the tea juices and reference infusion.

TABLE 2

| Component | Juice A | Juice B | Juice C | Ref. Infusion |
|---|---|---|---|---|
| TF1 (mg/ml) | 0.271 | 0.251 | 0.150 | 0.019 |
| TF2 (mg/ml) | 0.119 | 0.126 | 0.074 | 0.017 |
| TF3 (mg/ml) | 0.200 | 0.098 | 0.057 | 0.013 |
| TF4 (mg/ml) | 0.093 | 0.076 | 0.040 | 0.012 |
| Catechins (mg/ml) | 20.831 | 0.338 | 0.000 | 0.169 |
| Total Polyphenols (mg/ml) | 35.88 | 14.49 | 12.33 | 0.793 |
| Theanine (mg/ml) | 2.130 | 2.120 | 2.135 | 0.0526 |
| Caffeine (mg/ml) | 4.587 | 1.726 | 1.582 | 0.274 |
| TF1/TF4 | 2.93 | 3.29 | 3.73 | 1.50 |
| %TF1 in Theaflavins | 39.7 | 45.5 | 46.7 | 30.6 |
| %Catechins in Polyphenols | 58.1 | 2.33 | 0.00 | 21.4 |
| Theanine/Caffeine | 0.46 | 1.23 | 1.35 | 0.19 |

This data shows that the amount of caffeine and gallated theaflavins in tea juice decreases the later in the fermentation process that the juice is expressed.

Example 2

This Example demonstrates the processing of fresh leaf to produce black leaf tea and black tea juice.

*Production of Juice*

Fresh tea leaves (which had not been withered) were chopped using a vegetable cutter before being fed through a CTC (cut, tear, curl) machine (Rollers set at six teeth per inch with speeds of 1000 and 100 rpm respectively). The fresh dhool was then fermented for 2 hours at 25°C using a Teacraft™ fermentation unit (0.5°C wet bulb depression, 90 % R.H.) .
Fermented dhool was then pressed using a hydraulic press (5 Tonnes applied to a 500 g mass of fermented leaf inside a cylinder of diameter 160 mm, resulting in a downward pressure of 354 psi (2.44 MPa)) to express black tea juice. The yield of black tea juice was 25 ml/100 g fermented dhool, and had a total solids content of 8% by weight. The tea juice was immediately centrifuged for 20 minutes (10000 g at 3°C) and the supernatant was then filter sterilised using a Nalgene™ filtration unit fitted with a 0.2 $\mu$m filter. The solids content of the tea juice after centrifugation and filtration was 6% by weight. Examples of levels of tea actives in black tea juice are shown in table 3.

TABLE 3

| Tea active | Level in Black Juice ($\mu$g ml$^{-1}$) |
|---|---|
| Catechins | 0 |
| Theaflavins | 330 |
| Theanine | 2040 |
| Caffeine | 1465 |

*Production of Leaf Tea*

The pressed residual dhool resulting from the juice production above was broken up by hand and then dried using a fluidized bed drier (ten minutes at 120°C, followed by ten minutes at 90°C) to obtain a made black tea with moisture content of 3% by weight.

The dried fermented dhool was used to make a high quality black tea infusion by infusing in freshly-boiled water (2 minutes at 1 % w/v and without agitation). The colour profile of the infusion was measured, and found to be comparable to a control black leaf tea made using the above process but with the pressing step omitted. Further similarities between the infusion made from pressed dried residual dhool and control black leaf tea were apparent following quantification of the non-volatile tea components. The details shown in table 4 illustrate the comparable non-volatile profiles between the two, indicative of a good quality infusion despite being made from juice-extracted (i.e. pressed) dhool.

TABLE 4

| Tea component | Concentration in infusion of tea from residual dhool ($\mu$g ml$^{-1}$) | Concentration in control black tea infusion ($\mu$g ml$^{-1}$) |
|---|---|---|
| Catechins | 115 | 123 |
| Theaflavins | 57 | 51 |
| Theanine | 33 | 46 |
| Caffeine | 314 | 304 |

*Processing of Tea Juice*

The expressed black tea juice described above is a useful raw material for the production of freeze dried powders enriched in tea actives. As stated, the black tea juice has a total solids content of 6% post filtration, and this juice can be freeze dried to produce a powder of composition shown in table 5. The complete tea juice (i.e. freeze dried with no fractionation) produced a low-caffeine tea powder suitable as a base for ready-to-drink beverages or as a supply of actives in other products.

TABLE 5

| Tea component | Level in powder (mg g$^{-1}$ dry weight) |
|---|---|
| Catechins | 0 |
| Theaflavins | 5 |
| Theanine | 33 |
| Caffeine | 23 |

Example 3

This Example demonstrates the production of a ready-to-drink tea-based beverage from black tea juice.

Fresh tea leaves were chopped using a vegetable cutter, passed twice through a CTC machine and then fermented for 2 hours. The fermented dhool was pressed at 5 tonne pressure using a static press to yield the black juice.

The juice was clarified using a Beckman Avanti J-25 centrifuge (JLA-9.1000 rotor, 10 minutes at 12500 rcf, 20°C). The clarified juice contained 6.5 wt% tea solids. The juice was formulated into a beverage by combining the ingredients shown in table 6.

TABLE 6

| Ingredient | Amount (g/l) |
|---|---|
| Tea Juice | 30.8 |
| Sodium Bicarbonate | 0.1 |
| Ascorbic Acid | 0.2 |
| Deionised Water | 968.9 |

The product was UHT-treated (30 seconds at 136°C) cooled to 80°C and then filled and sealed into 150 ml cans. The cans were inverted for 1 minute to pasteurise the lids and then quickly cooled to 20°C in a water bath.

The resulting RTD beverage was found to have a caffeine content of 5.5 mg/100 ml and a theanine content of 6.2 mg / 100 ml. The weight ratio of TF1 to TF4 was much greater than 2.0.

## Claims

1. A process comprising the steps of:

   a) providing fresh tea leaves comprising catechins;
   b) macerating the fresh tea leaves thereby to produce dhool;
   c) fermenting the dhool for a fermentation time ($t_F$) sufficient to reduce the content of catechins in the dhool to less than 50% of the content of catechins in the fresh tea leaves prior to maceration on a dry weight basis; and then
   d) expressing juice from the fermented dhool thereby to produce leaf residue and tea juice, wherein the amount of expressed juice is at least 50 ml per kg of the fresh tea leaves.

2. A process according to claim 1 wherein $t_F$ is sufficient to reduce the amount of catechins in the dhool to less than 40% of the content of catechins in the fresh tea leaves on a dry weight basis.

3. A process according to claim 1 or claim 2 wherein $t_F$ is at least 1 hour, preferably between 1.5 and 24 hours.

4. A process according to any one of the preceding claims wherein the amount of expressed juice in step (d) is at least 100 ml per kg of the fresh tea leaves, preferably between 150 and 800 ml.

5. A process according to any one of the preceding claims wherein the process comprises the additional step of (e) processing the leaf residue to produce leaf tea.

6. A process according to any one of the preceding claims wherein the tea juice is used to make a tea product wherein the tea juice is the only source of tea solids in the tea product.

7. Tea juice obtainable by the process according to any one of the preceding claims.

8. A beverage obtainable by diluting the tea juice of claim 7 with an aqueous liquid.

9. A tea juice according to claim 7 or beverage according to claim 8 and comprising polyphenols wherein the polyphenols comprise catechins in an amount of less than 50% by weight of the polyphenols, preferably less than 40%.

10. A tea juice or beverage according to any one of claims 7 to 9 and comprising theaflavins wherein the theaflavins comprise theaflavin (TF1) and theaflavin digallate (TF4) and wherein the weight ratio of theaflavin to theaflavin digallate (TF1/TF4) is at least 2.0, preferably at least 3.0.

11. A tea juice or beverage according to any one of claims 7 to 10 and comprising theanine and caffeine wherein the weight ratio of theanine to caffeine is greater than 0.7, preferably at least 1.0.

## Patentansprüche

1. Verfahren, umfassend die Schritte:

   a) Bereitstellen von frischen Teeblättern, die Catechine umfassen;
   b) Mazerieren der frischen Teeblätter, um dadurch Dhool herzustellen;
   c) Fermentieren des Dhools für eine Fermentationszeit ($t_F$), die ausreichend ist, um den Gehalt an Catechinen in dem Dhool auf weniger als 50 % des Gehalts an Catechinen in den frischen Teeblättern vor Mazeration, auf Trockengewichtsbasis, zu verringern, und danach
   d) Auspressen von Saft aus dem fermentierten Dhool, um dadurch Blätterrückstand und Teesaft herzustellen, wobei die Menge an ausgedrücktem Saft wenigstens 50 ml pro kg der frischen Teeblätter ist.

**2.** Verfahren gemäß Anspruch 1, wobei $t_F$ ausreichend ist, um die Menge an Catechinen in dem Dhool auf weniger als 40 % des Gehalts an Catechinen in den frischen Teeblättern, auf Trockengewichtsbasis, zu verringern.

**3.** Verfahren gemäß Anspruch 1 oder Anspruch 2, wobei $t_F$ wenigstens 1 Stunde, vorzugsweise zwischen 1,5 und 24 Stunden, ist.

**4.** Verfahren gemäß einem der vorangehenden Ansprüche, wobei die Menge an ausgepresstem Saft in Schritt (d) wenigstens 100 ml pro kg der frischen Teeblätter, vorzugsweise zwischen 150 und 800 ml, ist.

**5.** Verfahren gemäß einem der vorangehenden Ansprüche, wobei das Verfahren den zusätzlichen Schritt (e) Prozessieren des Blätterrückstands unter Herstellung von Blatttee umfasst.

**6.** Verfahren gemäß einem der vorangehenden Ansprüche, wobei der Teesaft verwendet wird, um ein Teeprodukt herzustellen, wobei der Teesaft die einzige Quelle für Teefeststoffe in dem Teeprodukt ist.

**7.** Teesaft, der durch das Verfahren gemäß einem der vorangehenden Ansprüche erhältlich ist.

**8.** Getränk, das durch Verdünnen des Teesafts gemäß Anspruch 7 mit einer wässrigen Flüssigkeit erhältlich ist.

**9.** Teesaft gemäß Anspruch 7 oder Getränk gemäß Anspruch 8, der/das Polyphenole umfasst, wobei die Polyphenole Catechine in einer Menge von weniger als 50 Gewichts-% der Polyphenole, vorzugsweise weniger als 40 %, umfassen.

**10.** Teesaft oder -getränk gemäß einem der Ansprüche 7 bis 9, der/das Theaflavine umfasst, wobei die Theaflavine Theaflavin (TF1) und Theaflavindigallat (TF4) umfassen und wobei das Gewichtsverhältnis von Theaflavin zu Theaflavindigallat (TF1/TF4) wenigstens 2,0, vorzugsweise wenigstens 3,0, ist.

**11.** Teesaft oder -getränk gemäß einem der Ansprüche 7 bis 10, der/das Theanin und Coffein umfasst, wobei das Gewichtsverhältnis von Theanin zu Coffein größer als 0,7, vorzugsweise wenigstens 1,0, ist.


**Revendications**

**1.** Procédé comprenant les étapes suivantes :

a) l'obtention de feuilles fraîches de thé comprenant des catéchines ;
b) la macération des feuilles fraîches de thé pour ainsi produire un dhool ;
c) la fermentation du dhool pendant une durée de fermentation ($t_F$) suffisante pour réduire la teneur en catéchines dans le dhool à moins de 50 % de la teneur en catéchines dans les feuilles fraîches de thé avant la macération, sur une base de poids sec ; puis
d) l'expression d'un jus à partir du dhool fermenté, pour ainsi produire un résidu de feuille et un jus de thé, la quantité de jus exprimé étant d'au moins 50 ml par kg de feuilles fraîches de thé.

**2.** Procédé selon la revendication 1, dans lequel $t_F$ est suffisante pour réduire la quantité de catéchines dans le dhool à moins de 40 % de la teneur en catéchines dans les feuilles fraîches de thé, sur une base de poids sec.

**3.** Procédé selon la revendication 1 ou la revendication 2, dans lequel $t_F$ est au moins 1 heure, de préférence entre 1,5 heure et 24 heures.

**4.** Procédé selon l'une quelconque des revendications précédentes, dans lequel la quantité de jus exprimé dans l'étape (d) est au moins de 100 ml par kg de feuilles fraîches de thé, de préférence entre 150 ml et 800 ml.

**5.** Procédé selon l'une quelconque des revendications précédentes, dans lequel le procédé comprend l'étape (e) supplémentaire suivante : le traitement du résidu de feuille pour produire un thé en feuilles.

**6.** Procédé selon l'une quelconque des revendications précédentes, dans lequel le jus de thé est utilisé pour préparer un produit de thé dans lequel le jus de thé est la seule source de solides de thé dans le produit de thé.

7. Jus de thé pouvant être obtenu par le procédé selon l'une quelconque des revendications précédentes.

8. Boisson pouvant être obtenue par la dilution du jus de thé selon la revendication 7 avec un liquide aqueux.

9. Jus de thé selon la revendication 7 ou boisson selon la revendication 8, comprenant des polyphénols, les polyphénols comprenant des catéchines en une quantité inférieure à 50 % en poids des polyphénols, de préférence inférieure à 40 %.

10. Jus de thé ou boisson selon l'une quelconque des revendications 7 à 9, comprenant des théaflavines, les théaflavines comprenant de la théaflavine (TF1.) et du digallate de théaflavine (TF4) et le rapport pondéral entre la théaflavine et le digallate de théaflavine (TF1/TF4) étant d'au moins 2,0, de préférence d'au moins 3,0.

11. Jus de thé ou boisson selon l'une quelconque des revendications 7 à 10, comprenant de la théanine et de la caféine, le rapport pondéral entre la théanine et la caféine étant supérieur à 0,7, de préférence d'au moins 1,0.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4051264 A
- US 20070231445 A1
- GB 1284721 A
- GB 968423 A, A.M.H. Bake
- US 2007071870 A
- WO 2008040627 A
- WO 9940799 A
- EP 0167399 A
- WO 200047056 A
- WO 200182713 A
- WO 2003022066 A
- EP 1365657 B1
- CN 1718030 A

### Non-patent literature cited in the description

- Tea - Cultivation to consumption. Chapman & Hal, 1992, 555-601